# EUROPÄISCHE PATENTSCHRIFT

(11) **EP 2 291 432 B1**
(45) Veröffentlichungstag und Bekanntmachung des Hinweises auf die Patenterteilung: **15.08.2012**
(21) Anmeldenummer: 09765773.8
(22) Anmeldetag: 10.06.2009
(51) Int. Cl.: C08G 63/16

(54) **VERWENDUNG EINES C11-DIOLS ODER C11-DIOLGEMISCHES ZUR HERSTELLUNG VON POLYMEREN**
USE OF A C11 DIOL OR C11 DIOL MIXTURE FOR PRODUCING POLYMERS
UTILISATION D'UN DIOL EN C11 OU D'UN MÉLANGE DE DIOLS EN C11 POUR LA FABRICATION DE POLYMÈRES

(30) Priorität: 16.06.2008 EP 08158308
(43) Veröffentlichungstag der Anmeldung: 09.03.2011
(73) Patentinhaber: BASF SE, 67056 Ludwigshafen (DE)
(72) Erfinder: MIJOLOVIC, Darijo, 68309 Mannheim (DE); GARNIER, Sebastien, 69469 Weinheim (DE); MIAO, Qiang, 68199 Mannheim (DE); GUIXA GUARDIA, Maria, 68163 Mannheim (DE); TEBBEN, Gerd-Dieter, 68165 Mannheim (DE); WIEBELHAUS, Dag, 67435 Neustadt (DE)
(86) Internationale Anmeldenummer: PCT/EP2009/057133
(87) Internationale Veröffentlichungsnummer: WO 2009/153193

(56) Entgegenhaltungen:
- US-A1- 2008 118 855
- DATABASE WPI Week 199134 Thomson Scientific, London, GB; AN 1991-248700 XP002538818 & JP 03 161452 A (CHISSO CORP) 11. Juli 1991 (1991-07-11) in der Anmeldung erwähnt

## Beschreibung

Die Erfindung betrifft ein Polymer, welches erhältlich ist durch Polykondensation oder Polyadduktbildung von monomeren Verbindungen, wobei als monomere Verbindung
2-(2-Methyl) butyl-2-propyl-1,3propandiol der Formel I 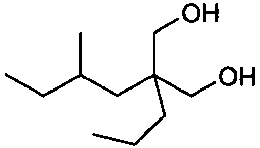 oder dessen alkoxylierte Derivate(im Nachfolgenden zusammenfassend auch als C11-Diol bezeichnet) mitverwendet wird.

Diole werden für die Herstellung von Polymeren, z.B. Polyestern oder Polyurethanen, benötigt. In EP-A 562 578 wird z. B. die Verwendung verschiedener Cyclohexandiole wie 1,4-Cyclohexandimethanol oder 1,4-Cyclohexandiethanol zur Herstellung von Polyestern beschrieben.

Die Verwendung von 2-Pentyl-2-propyl-1,3 propandiol der Formel II für die Herstellung von Polyestern ist aus JP HEI 03-161452 bekannt. Aus US 2008/118855 A1 sind Polyester bekannt, welche aus 1,2 Propandiol-Einheiten aufgebaut sind.

Grundsätzlich ist gewünscht, die anwendungstechnischen Eigenschaften von Polymeren bei ihren unterschiedlichen Verwendungen zu verbessern.

Bei einer Verwendung der Polymeren als Bindemittel in Beschichtungsmassen, Klebstoffen oder Dichtungsmassen ist insbesondere die Viskosität von Bedeutung sei es als Schmelzeviskosität (100% Systeme) oder als Lösungsviskosität (Polymerlösungen). Die hergestellten Beschichtungen sollen für Lackanwendungen gute mechanische Eigenschaften, wie Schlagzähigkeit und Elastizität, eine hohe Kratz- und Stoßfestigkeit, gute Beständigkeiten gegen Wasser, Lösemittel, Fett und Chemikalien und Umwelteinflüsse haben, sowie einen hohen Glanz aufweisen.

Aufgabe der vorliegenden Erfindung war, derartige Polymere zur Verfügung zu stellen.

Demgemäß wurden die eingangs definierten Polymere sowie ihre Verwendung als Bindemittel in Beschichtungsmassen, Dichtungsmassen oder Klebstoffen gefunden. Zum C11 Diol oder C11-Diolgemisch

Zur Herstellung des erfindungsgemäßen Polymeren wird, neben anderen monomeren Verbindungen, 2-(2-Methyl) butyl-2-propyl-1,3 propandiol der Formel I 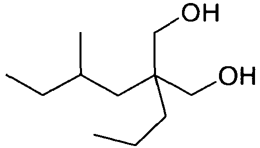 mitverwendet.

Im Nachfolgenden sollen bei 2-(2-Methyl) butyl-2-propyl-1,3 propandiol der Formel I und auch bei dem nachstehenden 2-Pentyl-2-propyl-1,3 propandiol der Formel II immer auch die alkoxylierten Derivate umfasst sein. Die Diole können insbesondere mit Ethylenoxid oder Propylenoxid oder auch deren Gemische alkoxyliert sein; Die Alkoholgruppen können z.B. mit 1 bis 20, insbesondere 1 bis 10 Alkoxygrupen alkoxyliert sein.

In einer bevorzugten Ausführungsform sind die beiden Diole nicht alkoxyliert.

Vorzugsweise werden neben 2-(2-Methyl) butyl-2-propyl-1,3 propandiol der Formel I im Gemisch mit 2-Pentyl-2-propyl-1,3 propandiol der Formel II

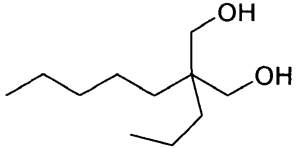

Als monomere Verbindung zur Herstellung der erfindungsgemäßen Polymere verwendet.

2-(2-Methyl) butyl-2-propyl-1,3 propandiol der Formel I wird im Nachfolgenden auch als C11 Diol; 2-(2-Methyl) butyl-2-propyl-1,3 propandiol und 2-Pentyl-2-propyl-1,3 propandiol werden im Nachfolgenden auch als C11-Diolgemisch bezeichnet.

Das C11-Diolgemisch kann z.B.
1 bis 99 Gew.-%, insbesondere 5 bis 95 Gew.-% 2-(2-Methyl) butyl-2-propyl-1,3 propandiol und
1 bis 99 Gew.-%, insbesondere 5 bis 95 Gew.-% 2-Pentyl-2-propyl-1,3 propandiol, enthalten, wobei sich die Gewichtsprozente auf die Gewichtssumme der genannten Diole beziehen.

Das C11-Diolgemisch enthält bevorzugt
1 bis 50 Gew.-%, insbesondere 2 bis 30 Gew.-%, besonders bevorzugt 5 bis 20 Gew. % und ganz besonders bevorzugt 7 bis 15 Gew. % 2-(2-Methyl) butyl-2-propyl-1,3 propandiol und entsprechend
50 bis 99 Gew.-%, insbesondere 70 bis 98 Gew.-% , besonders bevorzugt 80 bis 95 Gew. % und ganz besonders bevorzugt 85 bis 93 Gew. % 2-Pentyl-2-propyl-1,3 propandiol,
wobei sich die Gewichtsprozente auf die Gewichtssumme der beiden genannten Diole beziehen.

Ganz besonders geeignete C11-Diolgemische enthalten 2-Pentyl-2-propyl-1,3 propandiol und 2-(2-Methyl) butyl-2-propyl-1,3 propandiol in einem Gewichtsverhältnis von ca 8 bis 12 :1, d.h. die 8 bis 12fache Menge, insbesondere ca. die 10fache Menge 2-Pentyl-2-propyl-1,3 propandiol im Vergleich zum 2-(2-Methyl) butyl-2-propyl-1,3 propandiol.

Das C11- Diolgemisch kann weitere Bestandteile enthalten, die vorstehenden Gewichtsangaben sollen nur das Gewichtsverhältnis definieren, in dem die beiden Diole zueinander stehen.

### Zur Herstellung des C11-Diols oder C11-Diogemisches

2-Pentyl-2-propyl-1,3 propandiol und/ oder 2-(2-Methyl) butyl-2-propyl-1,3 propandiol können nach Verfahren des Stands der Technik hergestellt werden.

Im Falle des C11-Diolgemischs können die beiden Diole z.B. einzeln synthetisiert und anschließend in den gewünschten Verhältnissen gemischt werden oder aber auch unabhängig, ohne vorherige Mischung, für die Herstellung der Polymeren verwendet werden; wesentlich ist im Falle des C11-Diolgemisches, dass das Polymer beide Diole in den entsprechenden Mengen enthält.

Vorzugsweise wird das C11-Diolgemisch vorab als Gemisch hergestellt und als Gemisch für die Herstellung der Polymeren verwendet.

Nachfolgend wird ein bevorzugter Syntheseweg für das C11-Diolgemisch beschrieben. Aus dem C11-Diolgemisch können die gewünschten Diole abgetrennt werden und so, falls gewünscht, 2-Pentyl-2-propyl-1,3 propandiol und/ oder 2-(2-Methyl) butyl-2-propyl-1,3 propandiol in reiner Form gewonnen werden oder beliebige Mischungen dieser beiden Diole hergestellt werden.

WO 03/018192 beschreibt die Herstellung eines C10 Gemisches, welches 2-Propylheptenal und 2-Propyl-4-methylhexenal (z. B. in Mengen von ca 10 : 1) enthält (Verfahrensschritte a) bis c) des beanspruchten Verfahrens). Durch Hydroformylierung von Buten wird ein Gemisch von Pentanal (n-Valeraldehyd) und, in geringeren Mengen, 2-Methyl-butanal erhalten. Durch anschließende Aldolkondensation und Wasserabspaltung entsteht ein Gemisch von 2-Propylheptenal und 2-Propyl-4-methylhexenal.

Das erhaltene Gemisch von 2-Propylheptenal und 2-Propyl-4-methylhexenal kann in einem ersten Verfahrensschritt zu den entsprechenden Alkanalen (2-Propylheptanal und 2-Propyl-4-methylhexanal) hydriert werden. Die Hydrierung kann in üblicher Weise durchgeführt werden. Ein geeignetes Hydrierverfahren ist z.B. in DE-A 19524971 beschrieben.

Bevorzugt ist eine katalytische Hydrierung unter Verwendung üblicher Hydrierkatalysatoren, z. B. Palladium/Al₂O₃.
Zur Hydrierung können z.B. auch Nickel-, Kupfer-, Kupfer/Nickel-, Kupfer/Chrom-, Kupfer/Chrom/Nickel-, Zink/Chrom-, Nickel/Molybdän-Katalysatoren verwendet werden. Die Katalysatoren können trägerfrei sein, oder die hydrieraktiven Stoffe bzw. ihre Vorläufer können auf Trägern, wie beispielsweise SiO₂ oder Al₂O₃, aufgebracht sein. Vorzugsweise handelt es sich um eine Flüssigphasenhydrierung. Die Hydrierung kann bei einem Druck von 1 bis 100 bar durchgeführt werden. Die Reaktionstemperaturen liegen bevorzugt im Bereich von 50-200°C bevorzugt bei 80 bis 150°C.

Das nach der Hydrierung erhaltene Gemisch von 2-Propylheptanal und 2-Propyl-4-methylhaxenal kann durch eine gekreuzte Cannizzaro-Reaktion (Cannizzaro-Reaktion unter Verwendung von zwei verschiedenen Aldehyden; hier Aldehyd 1: das vorstehende Gemisch und Aldehyd 2: Formaldehyd). Die Durchführung einer derartigen gekreuzten Cannizzaro-Reaktion ist dem Fachmann bekannt und z. B. in JP-HEI 03-161452 beschrieben. Als Katalysator wird vorzugsweise ein Alkalihydroxid (z.B. NaOH, Ca(OH)₂, KOH) verwendet. Bei der gekreuzten Cannizzaro-Reaktion bildet sich Ameisensäure, welche als Salz (z.B. Natriumformiat oder Kaliumformiat) anfällt und leicht abgetrennt werden kann.

Ein anderer Syntheseweg statt der gekreuzten Cannizzaro-Reaktion ist z. B. die Aldolreaktion von 2-Propylheptenal und 2-Propyl-4-methylhexenal mit Formaldehyd und anschließende Hydrierung zu dem C11-Diolgemisch, wie z. B. in WO 04/092097 beschrieben.

Das vorstehende Verfahren kann in allen Verfahrensstufen diskontinuierlich oder bevorzugt kontinuierlich durchgeführt werden.

Besonders bevorzugt ist ein C11-Diolgemisch, welches durch das vorstehende Verfahren, das heißt durch
a) Hydroformylierung von Buten zu Pentanal und 2-Methyl-butanal
b) anschließende Aldolreaktion unter Wasserabspaltung zu 2-Propylheptenal und 2-Propyl-4-methylhexenal I
c) anschließende Hydrierung zu 2-Propylheptanal und 2-Propyl-4-methylhexanal und
d) gekreuzte Cannizzaro-Reaktion von 2-Propylheptanal und 2-Propyl-4-methylhexanal und Formaldehyd oder, alternativ, Aldolreaktion von 2-Propylheptenal und 2-Propyl-4-methylhexenal mit Formaldehyd und anschließende Hydrierung
erhältlich ist.

Das so erhaltene C11-Diolgemisch kann gegebenenfalls noch weitere Bestandteile, insbesondere andere Diole oder noch Aldehyde enthalten.

Das erhaltene C11-Diolgemisch besteht im Allgemeinen zu mindestens 90 Gew.-% aus den beiden Diolen der Formel I and II.

Aus dem erhaltenen Gemisch können einzelne Diole durch Destillation abgetrennt werden oder die Konzentration der einzelnen Diole geändert und gezielt eingestellt werden.

Falls alkoxylierte Diole gewünscht sind, kann anschließend eine Alkoxylierung der Hydroxylgruppen mit Alkylenoxiden, wie Ethylenoxid oder Propylenoxid, durchgeführt werden

### Zu den Polymeren

Die Polymer sind erhältlich durch Polykondensation oder Polyadduktbildung von monomeren Verbindungen unter Mitverwendung des C11-Diols oder des C11-Diolgemischs; die Polymeren können, wenn gewünscht, durch andere oder weitere Umsetzungen chemisch modifiziert, z.B. funktionalisiert oder vernetzt werden.

Bei einer Polykondensation von monomeren Verbindungen kommt es zu einer Abspaltung von Wasser oder Alkohol, bei einer Polyadduktbildung kommt es nicht zu einer Abspaltung.

Bevorzugte Polykondensate sind Polyester, welche durch Umsetzung von Di- oder Polyolen mit Di- oder Polycarbonsäuren, welche auch in Form reaktiver Derivate, wie Anhydride oder Ester eingesetzt werden können, erhältlich sind.
Unter dem Begriff Polyester soll im Folgenden ein Polymer verstanden werden, welches zu mehr als 50 Gew.-%, besonders bevorzugt zu mehr als 70 Gew.-% und insbesondere zu mehr als 90 Gew.-% aus Aufbaukomponenten, ausgewählt aus Diolen, Polyolen, Dicarbonsäuren und Polycarbonsäuren, besteht.
Genannt seien auch Polycarbonatdiole, welche durch Umsetzung von Dialkylcarbonaten mit Diolen unter Abspaltung von Alkoholen erhältlich sind.

Als Polyaddukt sei insbesondere Polyurethan genannt. In Betracht kommen z.B. auch Polyaddukte, die durch ringöffnende Polymerisation von Lactonen oder Lactamen erhältlich sind.

Unter dem Begriff Polyurethan soll im Folgenden ein Polymer verstanden werden, welches zu mehr als 50 Gew.-%, besonders bevorzugt zu mehr als 70 Gew.-% uns insbesondere zu mehr als 90 Gew.-% aus Aufbaukomponenten, ausgewählt aus Diisocyanaten, Polyisocyanaten, Diolen und Polyolen, besteht.

All diesen Polymeren ist gemeinsam, dass sie im Wesentlichen aus Diolen und mit diesen Diolen reaktiven Verbindungen, wie Di- bzw. Polycarbonsäuren (Polyester) oder Di- bzw. Polyisocyanaten (Polyurethane) aufgebaut sind.

Bevorzugte Polymere sind Polyester und Polyurethane, besonders bevorzugt sind Polyester.

Die erfindungsgemäßen Polymere haben vorzugsweise den nachstehenden Gehalt des C11-Diols oder des C11-Diolgemisches; die nachstehenden Gewichtsangaben zum Gehalt des C11-Diols oder C11-Diolgemisches im Polymer beziehen sich dabei auf die Einheiten des Polymeren, die sich vom C11-Diol oder C11-Diolgemisch ableiten. Bei Polyaddukten entspricht das Gewicht dieser Einheiten unverändert dem C11-Diol oder C11-Diolgemisch, bei Polykondensaten ist das Gewicht dieser Einheiten um die Wasserstoffatome der Hydroxylgruppen vermindert.

Bevorzugte Polymere bestehen mindestens zu 0,5 besonders bevorzugt mindestens zu 2, ganz besonders bevorzugt zu mindestens 5 und insbesondere zu mindestens 10 Gew % und in einer besonderen Ausführungsform zu mindestens 20 Gew.-% aus dem C11-Diol oder dem C11-Diolgemisch. Da die Mitverwendung von anderen, mit den Diolen reaktiven Verbindungen zwingend ist, bestehen die Polymeren im Allgemeinen zu nicht mehr als 70 Gew.-% insbesondere zu nicht mehr als 60 Gew.-% bzw. zu nicht mehr als 50 Gew.-% aus dem C11-Diol oder dem C11-Diolgemisch.

Neben dem C11-Diol oder C11-Diolgemisch können die Polymeren auch andere Diole oder Polyole als Aufbaukomponenten enthalten. In einer bevorzugten Ausführungsform handelt es sich bei mindestens 10 Gew.-%, besonders bevorzugt bei mindestens 25 Gew.-% und ganz besonders bevorzugt bei mindestens 50 Gew.-% der Diole und Polyole, aus denen die Polymeren bestehen, um das C11-Diol oder das C11-Diolgemisch.

Insbesondere kann es sich bei mindestens 70 Gew.-% bzw. mindestens 90 Gew.-% der Diole und Polyole, aus denen die Polymeren bestehen, um das C11 Diol oder das C11-Diolgemisch handeln.

In einer besonderen Ausführungsform kann es sich bei 100 Gew.-% aller Diole und Poyole, aus denen die Polymeren bestehen, um das C11-Diol- oder das C11-Diolgemisch handeln.

### Zu weiteren Bestandteilen der Polyester

Polyester können neben dem C11-Diol oder C11-Diolgemisch weitere Diole oder Polyole als Aufbaukomponenten enthalten.

Als Diole seien z.B. Ethylenglykol, Propylenglykol, und deren höher kondensierte Vertreter, z.B. wie Diethylenglykol, Triethylenglykol, Dipropylenglykol, Tripropylenglykol etc., Butandiol, Pentandiol, Hexandiol, Neopentylglykol, alkoxylierte phenolische Verbindungen, wie ethoxylierte bzw. propoxylierte Bisphenole, Cyclohexandimethanol genannt; als weitere Aufbaukomponente geeignete Polyole sind trifunktionelle und höherfunktionelle Alkohole, wie Glycerin, Trimethylolpropan, Butantriol, Trimethylolethan, Pentaerythrit, Neopentylglycol, Ditrimethylolpropan, Dipentaerythrit, Sorbit, Mannit.

Die vorstehenden Diole oder Polyole können alkoxyliert, insbesondere ethoxy- und propoxyliert sein. Die Alkoxylierungsprodukte sind in bekannter Weise durch Umsetzung der vorstehenden Alkohole mit Alkylenoxiden, insbesondere Ethylen- oder Propylenoxid, erhältlich. Vorzugsweise beträgt der Alkoxylierungsgrad je Hydroxylgruppe 0 bis 10, d.h. 1 mol Hydroxylgruppe kann vorzugsweise mit bis zu 10 mol Alkylenoxiden alkoxyliert sein.

Die Polyester enthalten weiterhin Dicarbonsäuren oder Polycarbonsäuren als Aufbaukomponenten. Dicarbonsäuren oder Polycarbonsäuren können bei der Herstellung der Polyester auch in Form ihrer reaktiven Derivate, z.B. als Anhydride oder Ester eingesetzt werden. Geeignete Dicarbonsäuren sind Bernsteinsäure, Glutarsäure, Adipinsäure, Sebacinsäure, Isophthalsäure, Terephthalsäure, deren Isomere und Hydrierungsprodukte, wie Tetrahydrophthalsäure. In Betracht kommen auch Maleinsäure und Fumarsäure für ungesättigte Polyester.

Polyester können auch Monoalkohole oder Monocarbonsäuren als Bestandteil enthalten; durch Mitverwendung derartiger Verbindungen kann das Molekulargewicht eingestellt, bzw. begrenzt werden.

Um besondere Eigenschaften zu erreichen werden, können die Polyester besondere funktionelle Gruppen enthalten. Wasserlösliche oder wasserdispergierbare Polyester enthalten die notwendige Menge an hydrophilen Gruppen, z.B. Carboxylgruppen oder Carboxylatgruppen um eine Wasserlöslichkeit oder Wasserdispergierbarkeit zu erreichen. Vernetzbare Polyester, z.B. für Pulverlacke, enthalten funktionelle Gruppen, welche mit dem verwendeten Vernetzungsmittel eine Vernetzungsreaktion eingehen. Es kann sich dabei ebenfalls um Carbonsäuregruppen handeln, wenn eine Vernetzung mit Hydroxylgruppen enthaltenden Verbindungen, z.B. Hydroxyalkylamiden beabsichtigt ist. Bei den funktionellen Gruppen kann es sich auch um ethylenisch ungesättigte Gruppen, z.B. durch Modifizierung des Polyester mit ungesättigten Dicarbonsäuren (Maleinsäure) oder Umsetzung mit (Meth)acrylsäure, handeln; derartige Polyester sind strahlungshärtbar.

Ungesättigte Polyester können auch mit einfach oder auch mehrfach ethylenisch ungesättigten, radikalisch polymerisierbaren Verbindungen, wie Styrol, C1-C10 Alkylacrylaten, Dialkylacrylaten, z. B. das Diacrylat von Ethandiol oder Butandiol copolymerisiert werden. Der ungesättigte Polyester kann dazu im Gemisch mit den ethylenisch ungesättigten Monomeren verwendet werden, wie z. B. in WO 00/23495 und EP 1131372 beschrieben ist. Die vorstehenden ethylenisch ungesättigten Verbindungen dienen dabei gleichzeitig als Lösemittel (Reaktivverdünner), so dass das Gemisch vorzugsweise als Lösung der Polyester in diesen Verbindungen vorliegt. Das Gemisch kann z. B. als Beschichtungs - oder Imprägnierungsmittel, insbesondere auch zur Herstellung von Laminaten verwendet werden. Die Härtung kann thermisch oder photochemisch, in beiden Fällen gegebenenfalls auch unter Zusatz eines Initiators erfolgen.

### Zu weiteren Bestandteilen der Polyurethane

Polyurethane enthalten als wesentliche Aufbaukomponente Di- oder Polyisocyanate.

Insbesondere zu nennen sind Diisocyanate X(NCO)2, wobei X für einen aliphatischen Kohlenwasserstoffrest mit 4 bis 15 Kohlenstoffatomen, einen cycloaliphatischen oder aromatischen Kohlenwasserstoffrest mit 6 bis 15 Kohlenstoffatomen oder einen araliphatischen Kohlenwasserstoffrest mit 7 bis 15 Kohlenstoffatomen steht. Beispiele derartiger Diisocyanate sind Tetramethylendiisocyanat, Hexamethylendiisocyanat, Dodecamethylendiisocyanat, 1,4-Diisocyanatocyclohexan, 1-Isocyanato-3,5,5-trimethyl-5-isocyanatomethylcyclohexan (IPDI), 2,2-Bis-(4-isocyanatocyclohexyl)-propan, Trimethylhexandiisocyanat, 1,4-Diisocyanatobenzol, 2,4-Diisocyanatotoluol, 2,6-Diisocyanatotoluol, 4,4'-Diisocyanato-diphenylmethan, 2,4'-Diisocyanato-diphenylmethan, p-Xylylendiisocyanat, Tetramethylxylylendiisocyanat (TMXDI), die Isomeren des Bis-(4-isocyanatocyclohexyl)methans (HMDI) wie das trans/trans-, das cis/cis- und das cis/trans-Isomere sowie aus diesen Verbindungen bestehende Gemische.

### Derartige Diisocyanate sind im Handel erhältlich.

Als Gemische dieser Isocyanate sind besonders die Mischungen der jeweiligen Strukturisomeren von Diisocyanatotoluol und Diisocyanatodiphenylmethan von Bedeutung, insbesondere ist die Mischung aus 80 mol-% 2,4-Diisocyanatotoluol und 20 mol-% 2,6-Diisocyanatotoluol geeignet. Weiterhin sind die Mischungen von aromatischen Isocyanaten wie 2,4-Diisocyanatotoluol und/oder 2,6-Diisocyanatotoluol mit aliphatischen oder cycloaliphatischen Isocyanaten wie Hexamethylendiisocyanat oder IPDI besonders vorteilhaft, wobei das bevorzugte Mischungsverhältnis der aliphatischen zu aromatischen Isocyanate 4 : 1 bis 1 : 4 beträgt.

Als Diole bzw. Polyole, welche mit den Di- oder Polyisocyanaten umgesetzt werden, wird erfindungsgemäß das C11 Diol allein oder das C11-Diol im Gemisch mit anderen Di- oder Polyolen verwendet.
Bei Polyurethanen werden als Diole vorzugsweise auch Polyesterole, insbesondere Polyesterdiole eingesetzt. Derartige Polyesterole werden vorab durch Umsetzung von Di- oder Polyolen mit Di- oder Polycarbonsäuren erhalten (siehe obige Beschreibung der Polyester). Das C11 Diol kann in den Polyurethanen in Form derartiger Polyesterole enthalten sein. Als weitere Diole, Polyole kommen die oben genanten in Betracht, sei es als Aufbaukomponenten welche direkt mit den Di- oder Polyisocyanaten umgesetzt werden, sei es als Bestandteil der Polyesterdiole. Als Dicarbonsäuren oder Polycarbonsäuren für die Polyesterdiole kommen ebenfalls die oben genannten in Betracht.

Die Polyurethane können auch Monoalkohole oder Monoisocyanate als Bestandteile enthalten; durch Mitverwendung derartiger Verbindungen kann das Molekulargewicht eingestellt, bzw. begrenzt werden.

Um besondere Eigenschaften zu erreichen werden, können die Polyurethane besondere funktionelle Gruppen enthalten. Wasserlösliche oder wasserdispergierbare Polyurethane enthalten die notwendige Menge an hydrophilen Gruppen, z.B. Carboxylgruppen oder Carboxylatgruppen um eine Wasserlöslichkeit oder Wasserdispergierbarkeit zu erreichen. Als geeignete Aufbaukomponente sei z.B. Dimethylolpropionsäure genannt. Vernetzbare Polyurethane, enthalten funktionelle Gruppen, welche mit dem verwendeten Vernetzungsmittel eine Vernetzungsreaktion eingehen. Die Polyurethane können neben Urethangruppen insbesondere auch andere funktionelle Gruppen, z.B. Harnstoffgruppen enthalten, welche durch Umsetzung der Di- oder Polyisocyanate mit Aminoverbindungen, entstehen.

Die Polymere können, wenn gewünscht, bei oder insbesondere auch zu einem späteren Zeitpunkt, z.B. bei der Verwendung, durch andere oder weitere Umsetzungen chemisch modifiziert, z.B. funktionalisiert oder vernetzt werden.

Insbesondere können die Polymeren vernetzende Gruppen enthalten, die, sobald die notwendigen Bedingungen vorliegen, eine Vernetzungsreaktion eingehen. Die Polymeren können insbesondere auch im Gemisch mit Vernetzern verwendet werden, die zum gewünschten Zeitpunkt unter den notwendigen Bedingungen (insbesondere bei erhöhter Temperatur) eine Vernetzungsreaktion mit dem Polymer eingehen.

Nach der Reaktivität der Vernetzer unterscheidet man zwischen einkomponentigen (1 K-) und zweikomponentigen (2K-) Systemen. Bei 2K-systemen wird der Vernetzer erst kurz vor der späteren Verwendung zugegeben, bei 1 K-Systemen kann der Vernetzer frühzeitig zum System gegeben werden (latenter Vernetzer), die Vernetzung tritt erst bei den später eingestellten Bedingungen auf, z.B. bei der Entfernung von Lösemittel und/oder Temperaturerhöhung.

Übliche Vernetzer sind z.B. Isocyanate, Epoxide, Säureanhydride oder bei Polymeren mit radikalisch polymersierbaren ethylenisch ungesättigten Gruppen, auch ethylenisch ungesättigte Monomere wie Styrol.

### Zur Verwendung der Polymere

Die Polymeren eignen sich als Bestandteil von thermoplastischen Zusammensetzungen. Die Polymeren, z.B. Polyester oder Polyurethane haben dazu vorzugsweise ein ausreichend hohes Molekulargewicht, damit sie thermoplastische Eigenschaften haben.

Thermoplastische Zusammensetzungen werden im Allgemeinen zur Herstellung von Formkörpern verwendet, wobei übliche Verfahren wie Spritzguss, Extrusion oder Blasformen zur Anwendung kommen können.

Insbesondere eignen sich die Polymeren als Bestandteil von Beschichtungsmassen, Dichtungsmassen oder Klebstoffen.

Die Beschichtungsmassen, Dichtungsmassen oder Klebstoffe enthalten die erfindungsgemäßen Polymere vorzugsweise als Bindemittel. Sie können weitere Bindemittel und sonstige Additive, z.B. Antioxidantien, Stabilisatoren, Farbstoffe, Pigmente, Verlaufshilfsmittel, Verdicker oder Benetzungshilfsmittel enthalten.

Bei den Beschichtungsmassen, Dichtungsmassen oder Klebstoffe kann es sich um wässrige oder lösemittelhaltige Massen handeln. Bevorzugt sind wässrige Massen. Derartige Massen enthalten die erfindungsgemäßen Bindemittel vorzugsweise in Form von Lösungen oder Dispersionen in Wasser oder organischen Lösemitteln oder deren Gemische. Soweit erforderlich, enthalten die Polymeren zusätzliche funktionelle Gruppen, die eine Löslichkeit oder Dispergierbarkeit in Wasser oder organischen Lösemitteln, bevorzugt in Wasser, bewirken (siehe oben).

Bei den Beschichtungsmassen, Dichtungsmassen oder Klebstoffe kann es sich auch um Massen handeln, die weitgehend frei sind von Wasser oder organischen Lösemitteln (sogenannte 100 % systeme). Derartige Massen enthalten im Allgemeinen weniger als 10 Gew.-Teile Wasser oder sonstige organische Lösemittel (Siedepunkt kleiner 150°C, bei 1 bar), auf 100 Gew.-Teile der Massen. Besonders bevorzugt enthalten sie weniger als 2 Gew.-Teile, ganz besonders bevorzugt weniger als 1 Gew. teil, bzw. weniger als 0,5 Gew.-Teile Wasser oder sonstige organische Lösemittel (Siedepunkt kleiner 150°C, bei 1 bar), auf 100 Gew.-Teile der Massen.

Es kann sich dabei Massen handeln, welche bei Raumtemperatur noch fließfähig sind oder um Massen, welche z.B. als Pulver vorliegen und erst bei erhöhten Temperaturen verarbeitet werden.

Die Massen, insbesondere Beschichtungsmassen, können strahlungshärtbar sein bzw. als strahlungshärtbare Massen bzw. Beschichtungsmassen verwendet werden. Vorzugsweise enthalten sie dazu ein strahlungshärtbares erfindungsgemäßes Polymer, insbesondere einen strahlungshärtbaren Polyester (siehe oben). Die Strahlungshärtung kann mit energiereicher Strahlung, z.B. Elektronenstrahlung oder UV Licht erfolgen; bei Verwendung von UV Licht kann den Polymeren vorzugsweise ein Photoinitiator zugesetzt werden.

Eine bevorzugte Verwendung im Rahmen der vorliegenden Erfindung ist die Verwendung der erfindungsgemäßen Polymere als oder in Pulverlacken. Vorzugsweise werden Polyester als Pulverlack verwendet, welche vernetzbar sind.
In einer bevorzugten Ausführungsform wird der Pulverlack durch Mischen und Aufschmelzen des Polyesters, Vernetzers und weiterer Additive, z.B. Pigmente und Verlaufsmittel bei hohen Temperaturen hergestellt. Das Gemisch kann durch anschließende Extrusion und entsprechende Verarbeitung des Extrudats in Pulverform gebracht werden.

Der Pulverlack kann in üblicher Weise, z.B. auch elektrostatisch, auf die gewünschten Substrate; z.B. solche mit Metall-, Kunststoff- oder Holzoberflächen, beschichtet werden.

Die erfindungsgemäßen Polymere haben eine geringe Viskosität, sowohl eine geringe Schmelzviskosität (100 % Systeme) oder eine geringe Lösungsviskosität (Polymerlösungen). Die geringe Viskosität erlaubt eine einfache Handhabung, bewirkt gute Beschichtungseigenschaften und erlaubt höhere Feststoffanteile in Lösungen oder Dispersionen oder geringere Bindemittelanteile in pigmenthaltigen Massen.
Die erfindungsgemäßen Polymere sind insbesondere auch sehr hydrolysebeständig.

Die erfindungsgemäßen Polymere bewirken bei ihrer Verwendung in Beschichtungsmassen, Dichtungsmassen und Klebstoffen gute mechanische Eigenschaften; insbesondere die Beschichtungsmassen, z.B. Pulverlacke; haben eine hohe Schlagzähigkeit, gute Elastizität und einen guten Glanz.

### Beispiele

### Herstellung des C11-Diolgemisches

Selektivhydrierung von 2-Propylheptenal:2-Propyl-4-methylhexenal:
Als Ausgangslösung diente einen Isomerengemisch aus u.a. 2-Propylheptenal und 2-Propyl-4-methylhexenal (∼10:1) die aus einem C5-Aldehyde Isomerengemisch hergestellt wurde, wie in WO 2003 018192 A1 beschrieben.

Das Gemisch wurde als Hydrierzulauf (Gemisch enthielt 91% Gew. % C10-Aldehyde) in Rieselfahrweise bei 40 bar H₂-Druck in einen auf 105 °C beheizten Reaktor (14 I Festbettreaktor 70 mm Durchmesser) gefahren. Die Belastung betrug 0,1 kg_{Organik}/I_{KAT}*h. Ein Teil des Hydrieraustrags wurde dem Zulauf wieder zugemischt (Kreislauffahrweise). Das Verhältnis von Kreislauf zu Zulauf betrug 10:1. Ein Zulauf von 1,3 kg/h und 500 NI/h Frischgas wurden dem Reaktor zugefahren. Im Austrag wurden u. a. 10,7% 2-Propyl-4-methylhexanal, 78,7% 2-Propylheptanal und 2% C10-Alkohole gefunden.

### Gekreuzte Cannizzaro -Reaktion

In einem 5000ml Reaktionskolben HWS mit gekühlten Rückflusskühler, Flüssigvorlage, Kryostat und KPG-Rührer wurden 865 g einer Mischung aus 2-Propylheptanal und 2-Propyl-4-methylhexanal (Gemisch enthielt 87% C10 gesättigte Aldehyde), 1500 g Formaldehyd (30%ig in Wasser) und 238 g Methanol vorgelegt. In einem Zeitraum von 3 Stunden bei 50°C wurden mit einer HPLC-Pumpe 1273g 25%ige NaOH zugetropft. Der Ansatz wurde 1 h bei 55°C weitergerührt. Nach Abkühlung bei Raumtemperatur wurde der pH-wert mit Ameisesäure auf gestellt. Die Phasen wurden getrennt und die organische Phase von Leichtsieder befreit (20 mbar und 85°C) und anschließend über eine Füllkörperkolonne (250 mm lang, 40 mm Durchmesser) unter Vakuum destilliert. 780g (83% Ausbeute) einer Mischung (∼10:1, 96%ig) von 2-Propyl-2-(2-methylbutyl)-1,3-propandiol (93 %) und 2-Propyl-2-pentyl-1,3-propandiol (5,8 %) wurde erhalten. Die Hauptfraktion wurde bei einer Kopftemperatur zwischen 128-132°C (Sumpftemperatur 152-165°C) bei einem Druck von 1 mbar gewonnen. Die Struktur der Diole wurde mittels GC, GC-MS und NMR (¹H, ¹³C) bestimmt.

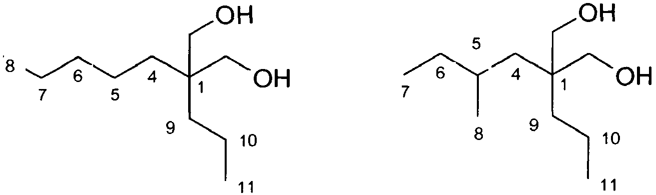

Chemische Verschiebungen bei ¹³C-NMR für die Hauptisomere (DMSO): 2-Propyl-2-pentyl-1,3-propandiol δ (ppm)= 64,1 (2 x CH₂OH), 21,9 (C₅), 22,2 (C₇), 32,5 (C₉), 14,9 (C₁₁), 41,1 (C₁), 30,4 (C₄), 33,0 (C₆), 13,83 (C₈), 15,6 (C₁₀); 2-Propyl-2-(2-methylbutyl)-1,3-propandiol δ (ppm)= 69,5 (2 x CH₂OH), 29,2 (C₅), 11,3 (C₇), 29,7 (C₉), 14,9 (C₁₁), 36,9 (C₁), 45,1 (C₄), 31,0 (C₆), 22,0 (C₈), 15,6 (C₁₀).

Herstellung einer Mischung ∼50:50 2-Propyl-2-(2-methylbutyl)-1,3-propandiol und 2-Propyl-2-pentyl-1,3-propandiol
Ein C11-Diolgemisch (10:90 2-Propyl-2-(2-methylbutyl)-1,3-propandiol und 2-Propyl-2-pentyl-1,3-propandiol) wurde an einer Füllkörperkolonne (2 m lang) destilliert. Bei 1 mbar Druck und einer Kopftemperatur zwischen 120-122 °C (Sumpftemperatur 172-178°C) wurde ein Gemisch der Diole von 50:50 gewonnen. Das Verhältnis der Diolen wurde mittels GC und NMR bestimmt.

Herstellung reines 2-Propyl-2-pentyl-1,3-propandiol (Reinheit 96 GC-FI%)
Dieses IDiol wurde durch Destillation einer Hauptfraktion des C11-Diolgemisches (-10:90 2-Propyl-2-(2-methylbutyl)-1,3-propandiol und 2-Propyl-2-pentyl-1,3-propandiol) an einer Füllkörperkolonne (2 m lang) gewonnen. Das reine 2-Propyl-2-pentyl-1,3-propandiol wurde bei 1 mbar Druck und 123°C Kopftemperatur (175-81°C) erhalten. Die Reinheit und Struktur wurde mittels GC und NMR bestimmt.

### Verwendungsbeispiele

### Beispiele

### Abkürzungen

ADS: Adipinsäure
D: Polydispersitätsindex (M_{w}/Mₙ)
DPG: Dipropylenglykol
DBZO: Dibutylzinoxid
DSC: Differential-Scanning-Kalorimetrie
GPC: Gelpermeationschromatographie
IPS: Isophtalsäure
Mₙ: zahlenmittleres Molekulargewicht in [g/mol]
M_{w}: gewichtsmittleres Molekulargewicht in [g/mol]
nFA: nicht-flüchtige Anteile
NPG: Neopentylglykol
OHZ: OH-Zahl
C11-Diolgemisch: Gemisch aus 2-Pentyl-2-propyl-1,3 propandiol und 2-(2-Methyl) butyl-2-propyl-1,3 propandiol (obige Formeln I und II) im Gewichtsverhältnis 10 : 1 PPPD: 2-Pentyl-2-propyl-1,2-propandiol
SZ: Säurezahl
T_{g}: Glasübergangstemperatur
TMP: Trimethylolpropan
TMSA: Trimellithsäureanhydrid
TPS: Terephtalsäure
η₁: Schmelzviskosität
η₂: Lösungsviskosität

### Polymercharakterisierungsmethoden

Die Molekulargewichtsbestimmungen werden mit GPC durchgeführt. Stationäre Phase: hochvernetztes poröses Polystyrol-Divinylbenzol, kommerziell erhältlich als PL-GEL von Fa. Polymer Laboratories. Laufmittel: THF. Fluss: 0,3 ml/min. Kalibrierung mit Polyethylenglykol 28700 bis 194 Dalton der Fa. PSS.
Die Säurezahl der Polyester wird nach der DIN-Norm-Methode 53169 bestimmt.
Die Bestimmung der Schmelzviskosität η₁ der Polyester wird mit einem Kegel-Platte-Viskosimeter bei 160°C im Oszillationsmodus und mit einer Winkelgeschwindigkeit von 0,1 rad/s durchgeführt. Die Bestimmung der Lösungsviskosität η₂ der Polyester wird mit einem Kegel-Platte-Viskosimeter bei Raumtemperatur im Rotationsmodus durchgeführt. Die Lösungen bestehen aus 70% Polyester und 30% Lösemittel (Mischung Solvesso 100T™ / Solvenon PM™ 5/1).
Die Tg der Polyester wird mittels DSC nach ASTM D3418 bestimmt.

### Herstellung Pulverpolyester mit COOH-Gruppen

### Polyester P1

### Stufe I - Herstellung des OH-Gruppen-haltigen Oligomers

149,1 g C11-Diolgemisch (0,80 Mol), 266,7 g NPG (2,56 Mol), 53,7 g TMP (0,4 Mol), 532,1 g TPS (3,21 Mol), und 0,7 g Katalysator DBZO werden in einem mit Thermometer, Schutzgaseinleitung, Rührer und Rückflusskühler ausgerüsteten 2L-Vierhalskolben vorgelegt. Unter Durchleiten eines Stickstoffstromes und unter Rückfluss wird die Reaktantenmischung auf 180°C zügig aufgeheizt. Wasser wird kontinuierlich abdestilliert. Anschließend wird das Reaktionsgemisch stufenweise auf 230°C innerhalb von 3 bis 5 St. unter Rühren und Stickstofffluss aufgeheizt, und bei 230°C weitergerührt, bis das Oligomer eine SZ von 10 bis 15 mg KOH/g aufweist. Die SZ des Oligomers beträgt 10 mg KOH/g.

### Stufe II - Herstellung des COOH-Gruppen-haltigen Polymers P1

Das oben synthetisierte Oligomer wird auf 180°C abgekühlt, bevor 133,0 g IPS (0,80 Mol) zugegeben werden. Die Temperatur wird auf 230°C erhöht, und es wird unter diesen Bedingungen weiterkondensiert, bis das Polymer eine SZ von 30 bis 40 mg KOH/g aufweist. Das aus der Polymerisation entstehende Wasser kann am Ende der Reaktion durch schwaches Vakuum gezogen werden, um die erwünschte SZ zu erreichen. Man erhält einen verzweigten COOH-Gruppen-haltigen Pulverpolyester P1, dessen SZ 30 mg KOH/g beträgt. P1 weist eine Glasüberganstemperatur T_{g} von 66 °C und eine Schmelzviskosität η₁ von 327 Pa.s bei 160°C auf. Die GPC-Analyse liefert folgende Werte: Mₙ= 2980 g/Mol ; D = 19,4 (siehe Tabelle 1).

### Polyester P2 bis P7

Es wird wie bei der Herstellung von P1 verfahren, mit den in Tabelle 1 zusammengefassten Zusammensetzungen. Man erhält verzweigte COOH-Gruppen-haltige Pulverpolyester, deren Kenndaten SZ, Mₙ, D, T_{g} und η₁ in Tabelle 1 aufgelistet sind.
P2 zum Vergleich

P3
P4
P5 zum Vergleich
P6
P7 zum Vergleich

**Tabelle 1**

| | Zusammensetzung | | | | | Polyesterkenndaten | | | | |
|---|---|---|---|---|---|---|---|---|---|---|
| Polyester | C11-Diol-gemisch [g] | NPG [g] | TMP [g] | TPS [g] | IPS [g] | SZ [mg KOH/g] | Mₙ [g/mol] | D | T_{g} [°C] | η₁ [Pa.s] |
| P1 | 149,1 | 266,7 | 53,7 | 532,1 | 133,0 | 30 | 280 | 19,4 | 66 | 327 |
| P2 | 0 | 374,9 | 57,5 | 570,0 | 142,5 | 36 | 2430 | 76,1 | 76 | 4660 |
| P3 | 152,2 | 283,9 | 15,8 | 475,5 | 203,8 | 57 | 1630 | 2,7 | 59 | 12,3 |
| P4 | 152,2 | 283,9 | 15,8 | 475,5 | 203,8 | 46 | 2270 | 3,3 | 67 | 41,5 |
| P5 | 0 | 396,0 | 17,0 | 510,3 | 218,7 | 57 | 1890 | 3,6 | 69 | 52,7 |
| P6 | 151,0 | 295,9 | 13,6 | 472,1 | 202,3 | 38 | 2525 | 3,5 | 67 | 42,8 |
| P7 | 0 | 407,9 | 14,6 | 506,1 | 216,9 | 38 | 2400 | 3,2 | 73 | 53,6 |

Die erfindungsgemäßen Polymere P1, P3 und P6 haben eine deutlich geringere Schmelzviskosität als die entsprechenden Vergleichspolymere P2, P5 und P7.

### Vergleichsbeispiel Polyester P8

Es wird wie bei der Herstellung von P1 verfahren, mit der in Tabelle 2 zusammengefassten Zusammensetzung. Polyester enthält kein C11-Diol-Gemisch, sonder das lineare PPPD.

**Tabelle 2**

| | Zusammensetzung | | | | | Polyesterkenndaten | | | | |
|---|---|---|---|---|---|---|---|---|---|---|
| Polyester | PPP D [g] | NPG [g] | TMP [g] | TPS [g] | IPS [g] | SZ [mg KOH/g] | Mₙ [g/mol] | D | T_{g} [°C] | η₁ [Pa.s] |
| P8 | 152,2 | 283,9 | 15,8 | 475,5 | 203,8 | 55 | 1940 | 3,3 | 66 | 33,6 |

Das erfindungsgemäß C11-Diol-Gemisch-haftige Polymer P3 (Tabelle 1) hat eine geringere Schmelzviskosität als das entsprechende PPPD-haltige Vergleichspolymer P8.

### Herstellung amorpher Polyester mit OH-Gruppen

### Polyester P9

206,25 g C11-Diolgemisch (1,11 Mol), 182,98 g NPG (1,76 Mol), 148,59 g TMP (1,11 Mol), 429,50 g IPS (2,59 Mol), 161,90 g ADS (1,11 Mol) und 0,5 g Katalysator DBZO werden in einem mit Thermometer, Schutzgaseinleitung, Rührer und Rückflusskühler ausgerüsteten 2L-Vierhalskolben vorgelegt. Unter Durchleiten eines Stickstoffstromes und unter Rückfluss wird die Reaktantenmischung auf 160°C zügig aufgeheizt. Wasser wird kontinuierlich abdestilliert. Anschließend wird das Reaktionsgemisch stufenweise auf 230°C innerhalb von 3 bis 5 St. unter Rühren und Stickstofffluss aufgeheizt, und bei 230°C weitergerührt, bis das Polyester P8 eine SZ von 10 bis 15 mg KOH/g aufweist. Man erhält einen verzweigten amorphen OH-Gruppen-haltigen Polyester P9, dessen SZ 14 mg KOH/g beträgt. P8 weist eine OHZ von 82 mg KOH/g und eine Glasüberganstemperatur T_{g} von 19 °C auf. Die GPC-Analyse liefert folgende Werte: Mₙ= 2000 g/Mol ; D = 5,3. P9 weist eine Schmelzviskosität η₁ von 1,3 Pa.s bei 160°C auf. Die Lösungsviskosität η₂ des Polyesters P8 bei Raumtemperatur (P8-Lösung mit 70% nFA und einer Mischung Solvesso 100™ / Solvenon PM™ 5/1 als Lösemittel) beträgt 12,1 Pa.s (siehe Tabelle 3).

### Polyester P10 (zum Vergleich)

Es wird wie bei der Herstellung von P9 verfahren, mit der in Tabelle 3 zusammengefassten Zusammensetzung. Die Kenndaten des Polyesters P10 sind in der Tabelle 3 aufgelistet.

**Tabelle 3**

| | Zusammensetzung | | | | | Polyesterkenndaten | | | | | | |
|---|---|---|---|---|---|---|---|---|---|---|---|---|
| Polyester | C11-Diol-gemisch [g] | NPG [g] | TMP [g] | IPS [g] | ADS [g] | SZ [mg KOH /g] | OHZ [mg KOH/g ] | Mₙ [g/mol] | D | Tg [°C] | η₁ [Pa.s] | η₂ [Pa.s] |
| P9 | 206,3 | 183,0 | 148,6 | 429,5 | 161,9 | 14 | 82 | 2000 | 5,3 | 19 | 1,3 | 12,1 |
| P10 | 0 | 326,6 | 163,9 | 473,9 | 178,6 | 15 | 108 | 2195 | 16,8 | 25 | 6,3 | 41,6 |

Das erfindunsgemäße Polymer P9 hat eine deutlich geringere Schmelzviskosität und eine deutlich geringere Lösungsviskosität als das Vergleichspolymer P10.

### Herstellung wasserverdünnbarer Polyester

### Polyester P11

### Stufe I - Herstellung des OH-Gruppen-haltigen Oligomers

242,8 g C11-Diolgemisch (1,29 Mol), 302,2 g NPG (2,91 Mol), 401,9 g IPS (2,42 Mol) und 0,5 g Katalysator DBZO werden in einem mit Thermometer, Schutzgaseinleitung, Rührer und Rückflusskühler ausgerüsteten 2L-Vierhalskolben vorgelegt. Unter Durchleiten eines Stickstoffstromes und unter Rückfluss wird die Reaktantenmischung auf 160°C zügig aufgeheizt. Wasser wird kontinuierlich abdestilliert. Anschließend wird das Reaktionsgemisch stufenweise auf 220°C innerhalb von 3 bis 5 St. unter Rühren und Stickstofffluss aufgeheizt, und bei 220°C weitergerührt, bis das Reaktionsgemisch eine SZ von 10 bis 15 mg KOH/g aufweist. Die SZ des Oligomers beträgt 15 mg KOH/g.

### Stufe II - Herstellung des Polymers P11

Das oben synthetisierte Oligomer wird auf 160°C abgekühlt, bevor 155,0 g TMSA (0,81 Mol) zugegeben werden. Die Temperatur wird auf 230°C erhöht, und es wird unter diesen Bedingungen weiterkondensiert, bis das Polymer eine SZ von 42 bis 48 mg KOH/g aufweist. Das aus der Polymerisation entstehende Wasser kann am Ende der Reaktion durch schwaches Vakuum gezogen werden, um die erwünschte SZ zu erreichen. Man erhält einen linearen wasserverdünnbaren Polyester P11, dessen SZ 46 mg KOH/g beträgt. P11 weist eine Glasüberganstemperatur T_{g} von 41 °C und eine Schmelzviskosität η₁ von 1,4 Pa.s bei 160°C auf. Die GPC-Analyse liefert folgende Werte: Mₙ= 1200 g/Mol ; D = 1,9 (siehe Tabelle 4).

### Beurteilung der Hydrolysenbeständigkeit von P11

Eine 20%-ige wässrige kolloidale Lösung von P11 wird hergestellt, auf pH 8 mit N,N-Dimethylethanolamin gebracht und bei 45°C gelagert. Das Zeitintervall bis die kolloidale Lösung ausfällt wird als Maß für die Hydrolysenbeständigkeit des Polyesters genommen (siehe Tabelle 5).

### Polyester P12 (zum Vergleich)

Es wird wie bei der Herstellung von P11 verfahren, mit der in Tabelle 4 zusammengefassten Zusammensetzung. Die Kenndaten des Polyesters P12 sind in der Tabelle 4 aufgelistet.

**Tabelle 4**

| | Zusammensetzung | | | | Polyesterkenndaten | | | | | |
|---|---|---|---|---|---|---|---|---|---|---|
| Polyester | C11-Diol-gemisch [g] | NPG [g] | IPS [g] | TMSA [g] | SZ [mg KOH/g] | OHZ [mg KOH/g] | Mn [g/mol] | D | T_{g} [°C] | η₁ [Pa.s] |
| P11 | 242,8 | 302,2 | 401,9 | 155,0 | 46 | 57 | 1200 | 1,9 | 41 | 1,4 |
| P12 | 0 | 490,4 | 451,6 | 174,1 | 47 | 58 | 1250 | 2,3 | 51 | 3,7 |

Das erfindungsgemäße Polymer P11 hat eine deutlich geringere Schmelzviskosität als das Vergleichspolymer P12.

**Tabelle 5**

| Polyester | Zeit bis Ausfallen der wässrigen Lösung (Tage) |
|---|---|
| P11 | > 30 Tage |
| P12 | 17 Tage |

Das erfindungsgemäße Polymer P11 ist deutlich hydrolysenbeständiger als das Vergleichspolymer P12.

### Herstellung Pulverlacke

Als Referenzbindemittel (REF) wird das Polyesterharz Uralac® P-862 (T_{g} 58,0 °C, SZ 35 mg KOH / g) von DSM Resins B.V. benutzt. Zur Herstellung der Pulverlacke PL3, PL4, PL5 und PLR werden entsprechend 570,0 g Pulverpolyester P3, P4, P5 (zum Vergleich) oder REF jeweils mit 30,0 g kommerziellem Härter Primid® XL-552 (Hydroxylalkylamid der Fa. DSM), 300,0 g Titandioxidpigment Kronos® 2160 (Fa. Kronos), 9,0 g Verlaufmittel Resiflow® PV5 (Fa. Worlée Chemie GmbH) und 2,5 g Entgasungsmittel Benzoin in einem Labor-Universalmischer (Fa. MIT Mischtechnik GmbH) vermischt, geschmolzen und anschließend in einem Doppelschnecken-Extruder (MP 19, Fa. APV) bei 80 - 100°C extrudiert. Das erhaltene Extrudat wird dann grob gebrochen, gemahlen und gesiebt. Die so erhaltenen Pulverlacke PL3, PL4 und PL5 werden folgende Prüfungen unterzogen:

| Prüfparameter | Prüfmethode |
|---|---|
| Fliesseigenschaften | Fluidisierbarkeit DIN ISO 8130-5 |
| | Tablettenablauf DIN ISO 8130-11 |
| Gelzeit | DIN ISO 8130-6 |

Im Anschluss werden die Pulverlacke auf Stahlprüfbleche (Q-Panel R-36) elektrostatisch appliziert und bei 160°C 10 Min. lang eingebrannt. Dabei werden Schichtdicken von 60 µm bis 80 µm angestrebt. Den resultierenden Beschichtungen werden folgende Prüfungen unterzogen:

| Prüfparameter | Prüfmethode |
|---|---|
| Erscheinungsbild | visuelle Beurteilung der Oberflächen |
| Glanz | DIN EN ISO 2813 |
| Schlagzähigkeit | EN ISO 6272 |
| Schlagempfindlichkeit | ASTM D 2794 |
| Elastizität | EN ISO 1520 |
| Wetterstabilität | Schnellbewitterungsprüfung (QUV-A) DIN EN ISO 11507 |

Die Ergebnisse der Lackprüfungen sind in der Tabelle 6 zusammengefasst.

**Tabelle 6**

| | Prüfparameter | Prüfverfahren | PL3 | PL4 | PL5 (Vergleich) | PLR (Vergleich) |
|---|---|---|---|---|---|---|
| | Fliesseigenschaften | Fluidisierbarkeit | 117,8 | 154,1 | 160,4 | 124,6 |
| Pulverlack | | Tablettenablauf @ 180°C [mm] | 22,2 | 18,8 | 13,5 | 30,5 |
| | Gelzeit | Gelzeit @ 180°C [s] | 195 | 154 | 154 | 173 |
| | Erscheinungsbild | Visuelle Beurteilung | 2* | 1** | 2 | 2 |
| | Glanz | Glanzmessung bei 20° | 81 | 79 | 75 | 63 |
| | Schlagzähigkeit | Reverse Impact [kg*cm] | 20 | 80 | 50 | 200 |
| Prüfbleche | Schlagempfindlichkeit | Impact [kg*cm] | 160 | 200 | 80 | 200 |
| | Elastizität | Erichsentiefung [mm] | 8,1 | 10,2 | 10,1 | 10,6 |
| | Wetterstabilität | Restglanz nach 1000 h QUV-A [%] | 82 | 86 | 84 | 88 |

| | | | | | | |
|---|---|---|---|---|---|---|
| * 2 = orange peel, pinholes ** 1 = orange peel, einzelne pinholes | | | | | | |

Die erfindungsgemäßen Pulverlacke haben sehr gute anwendungstechnische Eigenschaften. PL3 und PL4 haben einen besseren Glanz, PL4 hat bessere mechanische Eigenschaften als PL5.

### Herstellung festkörperreicher 1 Komponent-Lacke (1 K)

Zur Herstellung der festkörperreichen 1 K-Lacke 1 K-PL9 und 1 K-PL10 werden entsprechend 70%-ige Lösungen der Polyester P9 und P10 in Butylacetat hergestellt. 80 g der 70%-igen Polyesterlösungen werden jeweils mit 14 g kommerziellem Härter Luwipal® 066 (Melaminkondensat der Fa. BASF), 4 g n-Butanol, und 2 g Katalysator p-Toluolsulfonsäure vermischt. Die resultierenden Lösungen (nFA 70%) werden auf Glasplatten und Stahlprüfbleche mit Hilfe von Kastenrakel appliziert. Dabei werden Schichtdicken von 40 µm bis 50 µm angestrebt. Im Anschluss werden die beschichteten Prüfbleche bei 140°C 30 Min. lang eingebrannt. Den resultierenden Beschichtungen werden folgende Prüfungen unterzogen:

| | Prüfparameter | Prüfmethode |
|---|---|---|
| Glasplatten | Erscheinungsbild | visuelle Beurteilung der Oberflächen |
| | Glanz | DIN EN ISO 2813 |
| | Schlagempfi ndlichkeit | DIN 53157 |
| Stahlprüfbleche | Schlagempfindlichkeit | DIN 53157 |
| | Elastizität | DIN 53156 |
| | Hydrolysenbeständigkeit | Daimler-Chrysler-Test PBODCC371 |
| | Chemikalienbeständigkeit | Daimler-Chrysler-Test PBODCC371 |

Die Ergebnisse der Lackprüfungen sind in der Tabelle 7 zusammengefasst.
1 K-PL9 (auf Basis des Polyesters P9) ist erfindungsgemäß, 1 K-PL10 (auf Basis des Polyesters P10) gilt als Vergleichsbeispiel.

**Tabelle 7**

| | Prüfparameter | Prüfverfahren | 1K-PL9 | 1 K-PL10 |
|---|---|---|---|---|
| Glas-platten | Erscheinungsbild | Visuelle Beurteilung | klar | klar |
| | Glanz | Glanzmessung bei 20° | 166 | 175 |
| | Schlagempfindlichkeit | Pendeldämpfung (König) [Sekunden] | 220 | 232 |
| | | Pendeldämpfung [Ausschläge] | 164 | 166 |
| Stahl-prüf-bleche | Schlagempfindlichkeit | Pendeldämpfung (König) [Sekunden] | 224 | 227 |
| | | Pendeldämpfung [Ausschläge] | 160 | 164 |
| | Elastizität | Erichsentiefung [mm] | 8,5 | 8,3 |
| | Hydrolysenbeständigkeit | Tₘₐₓ [°C] - dest. Wasser | 63 | 78 |
| | Chemikalienbeständigkeit | Tₘₐₓ [°C] - Pancreatin in Wasser (50%) | 44 | 60 |
| | | Tₘₐₓ [°C] - Schwefelsäure (1%) | 53 | 39 |
| | | Tₘₐₓ [°C] - Natronlauge (1%) | 43 | 53 |

Der erfindungsgemäße festkörperreiche Lack 1 K-PL9 zeigt ein sehr gutes Eigenschaftsprofil. Insbesondere zeigt das C11-Diol-Gemisch einen Vorteil gegenüber NPG bei der Elastizität des Films, sowohl als auch bei der Säurenbeständigkeit.

### Herstellung festkörperreicher 2 Komponenten-Lacke (2K)

Zur Herstellung der festkörperreichen 2K-Lacke 2K-PL9 und 2K-PL10 werden entsprechend 70%-ige Lösungen der Polyester P9 und P10 in Butylacetat hergestellt. 70 g der 70%-igen Polyesterlösungen werden jeweils mit 1 g Lösung (10%-ig in Butylacetat) des Verlaufmittels Baysilon® OL17 (Polyether der Fa. Borchers GmbH), 1 g Katalysator Dibutylzinndilaurat-Lösung (5%-ig in Butylacetat), 3 g Methoxypropylacetat, 20 g kommerziellem Härter Basonat® HI 190 BS (90%-ig, Polyisocyanat der Fa. BASF) und 5 g Butylacetat vermischt. Die resultierenden Lösungen (nFA 67%) werden auf Glasplatten und Stahlprüfbleche mit Hilfe von Kastenrakel appliziert. Dabei werden Schichtdicken von 40 µm bis 50 µm angestrebt. Im Anschluss werden die beschichteten Prüfbleche bei 80°C 30 Min. lang eingebrannt. Den resultierenden Beschichtungen werden folgende Prüfungen unterzogen:

| | Prüfparameter | Prüfmethode |
|---|---|---|
| Glasplatten | Erscheinungsbild | visuelle Beurteilung der Oberflächen |
| | Glanz | DIN EN ISO 2813 |
| | Schlagempfindlichkeit | DIN 53157 |
| Stahlprüfbleche | Schlagempfindlichkeit | DIN 53157 |
| | Elastizität | DIN 53156 |
| | Hydrolysenbeständigkeit | Daimler-Chrysler-Test PBODCC371 |
| | Chemikalienbeständigkeit | Daimler-Chrysler-Test PBODCC371 |

Die Ergebnisse der Lackprüfungen sind in der Tabelle 8 zusammengefasst.

2K-PL9 (auf Basis des Polyesters P9) ist erfindungsgemäß, 2K-PL10 (auf Basis des Polyesters P10) gilt als Vergleichsbeispiel.

**Tabelle 8**

| | Prüfparameter | Prüfverfahren | 2K-PL9 | 2K-PL10 |
|---|---|---|---|---|
| Glas-platten | Erscheinungsbild | Visuelle Beurteilung | klar | klar |
| | Glanz | Glanzmessung bei 20° | 163 | 166 |
| | Schlagempfindlichkeit | Pendeldämpfung (König) [Sekunden] | 205 | 186 |
| | | Pendeldämpfung [Ausschläge] | 146 | 134 |
| Stahl-Prüfbleche | Schlagempfindlichkeit | Pendeldämpfung (König) [Sekunden] | 213 | 186 |
| | | Pendeldämpfung [Ausschläge] | 152 | 133 |
| | Elastizität | Erichsentiefung [mm] | 10,4 | 10,3 |
| | Hydrolysenbeständigkeit | Tmax [°C] - dest. Wasser | 50 | 55 |
| | Chemikalienbeständigkeit | Tₘₐₓ [°C] - Pancreatin in Wasser (50%) | 39 | 39 |
| | | Tₘₐₓ [°C] - Schwefelsäure (1%) | 49 | 50 |
| | | Tₘₐₓ [°C] - Natronlauge (1%) | 44 | 52 |

Der erfindungsgemäße festkörperreiche Lack 2K-PL9 zeigt ein sehr gutes Eigenschaftsprofil. Die mechanischen Eigenschaften sind deutlich besser als beim Lack 2K-PL10 auf Basis von NPG.

## Patentansprüche

1. Polymer, erhältlich durch Polykondensation oder Polyadduktbildung von monomeren Verbindungen, **dadurch gekennzeichnet, dass** als monomere Verbindung 2-(2-Methyl) butyl-2-propyl-1,3propandiol der Formel I 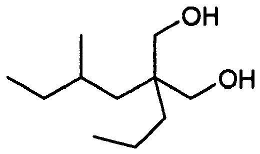 oder dessen alkoxylierte Derivate (im Nachfolgenden auch zusammenfassend C11-Diol genannt) mitverwendet wird.

2. Polymer gemäß Anspruch 1, **dadurch gekennzeichnet, dass** als monomere Verbindung ein Gemisch aus 2-(2-Methyl) butyl- 2-propyl-1,3 propandiol oder dessen alkoxylierten Derivate und 2-Pentyl-2-propyl-1,3 propandiol der Formel II 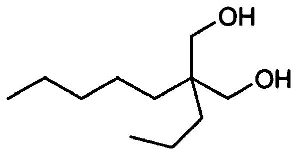 oder dessen alkoxylierten Derivate (im Nachfolgenden C11-Diolgemisch genannt) mitverwendet wird.

3. Polymer gemäß Anspruch 2, **dadurch gekennzeichnet, dass** das C11-Diolgemisch zu.
1 bis 99 Gew. % aus 2-(2-Methyl) butyl- 2-propyl-1,3 propandiol oder dessen alkoxylierten Derivate und zu
1 bis 99 Gew. % aus 2-Pentyl-2-propyl-1,3 propandiol oder dessen alkoxylierten Derivate besteht.

4. Polymer gemäß Anspruch 2 oder 3, **dadurch gekennzeichnet, dass** das C11-Diolgemisch durch
a) Hydroformylierung von Buten zu Pentanal und 2-Methyl-butanal
b) anschließende Aldolreaktion unter Wasserabspaltung zu 2-Propylheptenal und 2-Propyl-4-methylhexenal
c) anschließende Hydrierung zu 2-Propylheptanal und 2-Propyl-4-methylhexanal und
d) gekreuzte Cannizzaro Reaktion von 2-Propylheptanal und 2-Propyl-4-methylhexanal und Formaldehyd oder, alternativ, Aldolreaktion von 2-Propylheptenal und 2-Propyl-4-methylhexenal mit Formaldehyd und anschließende Hydrierung
erhältlich ist.

5. Polymer gemäß einem der Ansprüche 1 bis 4 **dadurch gekennzeichnet, dass** das Polymer zu 0,5 bis 70 Gew. % aus 2-(2-Methyl) butyl- 2-propyl-1,3 propandiol, dessen alkoxylierte Derivate (C11-Diol) oder dem C11-Diolgemisch besteht.

6. Polymer gemäß einem der Ansprüche 1 bis 5 **dadurch gekennzeichnet, dass** es sich um ein Polyester handelt.

7. Polymer gemäß einem der Ansprüche 1 bis 5, **dadurch gekennzeichnet, dass** es sich um ein Polycarbonatdiol (erhältlich durch Umsetzung von Dialkylcarbonaten mit Diolen unter Abspaltung von Alkohol) handelt.

8. Polymer gemäß einem der Ansprüche 1 bis 5, **dadurch gekennzeichnet, dass** es sich um ein Polyurethan handelt.

9. Polymer gemäß einem der Ansprüche 1 bis 5, **dadurch gekennzeichnet, dass** es um ein Polyaddukt handelt, welches durch ringöffnende Polymerisation von Lactonen oder Lactamen erhältlich ist.

10. Thermoplastische Zusammensetzungen, enthaltend ein Polymer gemäß einem der Ansprüche 1 bis 9.

11. Verwendung der thermoplastischen Zusammensetzungen gemäß Anspruch 10 zur Herstellung von Formkörpern.

12. Beschichtungsmassen, Dichtungsmassen oder Klebstoffe, enthaltend ein Polymer gemäß einem der Ansprüche 1 bis 9.

13. Beschichtungsmassen, Dichtungsmassen oder Klebstoffe gemäß Anspruch 12, **dadurch gekennzeichnet, dass** es sich um wässrige Massen handelt.

14. Pulverlack, enthaltend ein Polymer gemäß einem der Ansprüche 1 bis 9

15. Strahlungshärtbare Beschichtungsmassen, enthaltend ein Polymer gemäß einem der Ansprüche 1 bis 9.

## Claims

1. A polymer obtainable by polycondensation or polyaddition of monomeric compounds, wherefor accompanying use is made as monomeric compound of 2-(2-methylbutyl)-2-propyl-1,3-propanediol of the formula I 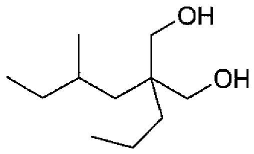 or its alkoxylated derivatives (also referred to collectively below as C11 diol).

2. The polymer according to claim 1, wherefor accompanying use is made as monomeric compound of a mixture of 2-(2-methylbutyl)-2-propyl-1,3-propanediol or its alkoxylated derivatives and 2-pentyl-2-propyl-1,3-propanediol of the formula II 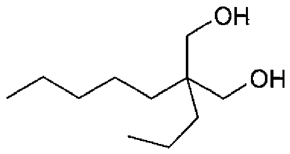 or its alkoxylated derivatives (referred to below as C11 diol mixture).

3. The polymer according claim 2, wherein the C11 diol mixture is composed of
1% to 99% by weight of 2-(2-methylbutyl)-2-propyl-1,3-propanediol or its alkoxylated derivatives and of
1% to 99% by weight of 2-pentyl-2-propyl-1,3-propanediol or its alkoxylated derivatives.

4. The polymer according to claim 2 or 3, wherein the C11 diol mixture is obtainable by
a) hydroformylation of butene to pentanal and 2-methylbutanal,
b) subsequent aldol reaction, with elimination of water, to 2-propylheptenal and 2-propyl-4-methylhexenal,
c) subsequent hydrogenation to 2-propylheptanal and 2-propyl-4-methylhexanal, and
d) crossed Cannizzaro reaction of 2-propylheptanal and 2-propyl-4-methylhexanal and formaldehyde or, alternatively, aldol reaction of 2-propylheptenal and 2-propyl-4-methylhexenal with formaldehyde and subsequent hydrogenation.

5. The polymer according to any of claims 1 to 4, wherein the polymer is composed of 0.5% to 70% by weight of 2-(2-methylbutyl)-2-propyl-1,3-propanediol, its alkoxylated derivatives (C11 diol) or the C11 diol mixture.

6. The polymer according to any of claims 1 to 5, which is a polyester.

7. The polymer according to any of claims 1 to 5, which is a polycarbonate diol (obtainable by reaction of dialkyl carbonates with diols, with elimination of alcohol).

8. The polymer according to any of claims 1 to 5, which is a polyurethane.

9. The polymer according to any of claims 1 to 5, which is a polyadduct which is obtainable by ring-opening polymerization of lactones or lactams.

10. A thermoplastic composition comprising a polymer according to any of claims 1 to 9.

11. The use of a thermoplastic composition according to claim 10 to produce a molding.

12. A coating composition, sealant or adhesive comprising a polymer according to any of claims 1 to 9.

13. The coating composition, sealant or adhesive according to claim 12, which is an aqueous composition.

14. A powder coating material comprising a polymer according to any of claims 1 to 9.

15. A radiation-curable coating composition comprising a polymer according to any of claims 1 to 9.

## Revendications

1. Polymère, pouvant être obtenu par polycondensation ou formation de produit de polyaddition de composés monomères, **caractérisé en ce qu'**on utilise conjointement comme composé monomère du 2-(2-méthyl)-butyl-2-propyl-1,3-propanediol de formule I 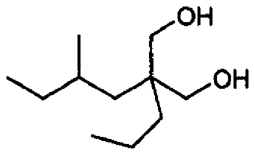 ou ses dérivés alcoxylés (également appelé C11-diol en résumé dans la suite).

2. Polymère selon la revendication 1, **caractérisé en ce qu'**on utilise conjointement comme composé monomère un mélange de 2-(2-méthyl)-butyl-2-propyl-1,3-propanediol ou ses dérivés alcoxylés et de 2-pentyl-2-propyl-1,3-propanediol de formule II 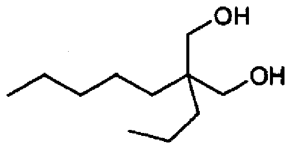 ou ses dérivés alcoxylés (également appelé mélange de C11-diols en résumé dans la suite).

3. Polymère selon la revendication 2, **caractérisé en ce que** le mélange de C11-diols est constitué, à raison de
- 1 à 99% en poids, de 2-(2-méthyl)-butyl-2-propyl-1,3-propanediol ou ses dérivés alcoxylés et, à raison de
- 1 à 99% en poids, de 2-pentyl-2-propyl-1,3-propanediol ou ses dérivés alcoxylés.

4. Polymère selon la revendication 2 ou 3, **caractérisé en ce que** le mélange de C11-diols peut être obtenu par
a) hydroformylation de butène en pentanal et en 2-méthylbutanal
b) réaction aldol consécutive avec dissociation d'eau en 2-propylhepténal et 2-propyl-4-méthylhexénal
c) hydrogénation consécutive en 2-propylheptanal et 2-propyl-4-méthylhexanal et
d) réaction croisée de Cannizzaro de 2-propylheptanal et de 2-propyl-4-méthylhexanal et de formaldéhyde ou, en variante, réaction aldol de 2-propylhepténal et 2-propyl-4-méthylhexénal avec du formaldéhyde et hydrogénation consécutive.

5. Polymère selon l'une quelconque des revendications 1 à 4, **caractérisé en ce que** le polymère est constitué, à raison de 0,5 à 70% en poids, de 2-(2-méthyl)-butyl-2-propyl-1,3-propanediol, ses dérivés alcoxylés (C11-diol) ou de mélange de C11-diols.

6. Polymère selon l'une quelconque des revendications 1 à 5, **caractérisé en ce qu'**il s'agit d'un polyester.

7. Polymère selon l'une quelconque des revendications 1 à 5, **caractérisé en ce qu'**il s'agit d'un polycarbonatediol (pouvant être obtenu par transformation de dialkylcarbonates avec des diols avec dissociation d'alcool).

8. Polymère selon l'une quelconque des revendications 1 à 5, **caractérisé en ce qu'**il s'agit d'un polyuréthane.

9. Polymère selon l'une quelconque des revendications 1 à 5, **caractérisé en ce qu'**il s'agit d'un produit de polyaddition qui peut être obtenu par polymérisation avec ouverture de cycle de lactones ou de lactames.

10. Compositions thermoplastiques contenant un polymère selon l'une quelconque des revendications 1 à 9.

11. Utilisation des compositions thermoplastiques selon la revendication 10 pour la production de corps façonnés.

12. Masses de revêtement, masses d'étanchéité ou adhésifs contenant un polymère selon l'une quelconque des revendications 1 à 9.

13. Masses de revêtement, masses d'étanchéité ou adhésifs selon la revendication 12, **caractérisés en ce qu'**il s'agit de masses aqueuses.

14. Laque en poudre, contenant un polymère, selon l'une quelconque des revendications 1 à 9.

15. Masses de revêtement durcissables par un rayonnement, contenant un polymère selon l'une quelconque des revendications 1 à 9.
